Europäisches Patentamt

European Patent Office

Office européen des brevets

(11) Veröffentlichungsnummer: **0 356 376**
A1

## EUROPÄISCHE PATENTANMELDUNG

(21) Anmeldenummer: **89810510.1**

(51) Int. Cl.⁵: **A 61 F 2/36**

(22) Anmeldetag: **06.07.89**

(30) Priorität: **15.08.88 CH 3061/88**

(43) Veröffentlichungstag der Anmeldung:
**28.02.90 Patentblatt 90/09**

(84) Benannte Vertragsstaaten: **AT DE FR GB IT**

(71) Anmelder: **GEBRÜDER SULZER
AKTIENGESELLSCHAFT
Zürcherstrasse 9
CH-8401 Winterthur (CH)**

(72) Erfinder: **Willert, Hans-Georg, Dr.-med.
Schlegelweg 9
D-3400 Göttingen (DE)**

Die Bezeichnung der Erfindung wurde geändert (Richtlinien für die Prüfung im EPA, A-III, 7.3).

(54) **Verankerungsschaft für eine Femurkopfprothese.**

(57) Das distale Ende eines Verankerungsschaftes (1) für eine Endoprothese ist zur Erhöhung der Elastizität mit einem Längsschlitz (2) versehen. Dieser ist durch ein Abschlusselement (3) nach distal geschlossen, um die Implantation des Schaftes (1) zu erleichtern, ohne die erhöhte Elastizität zu "vernichten".

Fig. 1

EP 0 356 376 A1

## Beschreibung

### Verankerungsschaft

Die Erfindung betrifft einen Verankerungsschaft für eine zementfreie Verankerung einer Femurkopfprothese, dessen distaler Bereich über eine gewisse Länge hohl ausgebildet oder mit einem Längsschlitz versehen ist.

Verankerungsschäfte der vorstehend genannten Art sind beispielsweise bekannt aus der DE-A-33 43 304 oder EP-A 222 336. Mit den Hohlräumen oder Schlitzen soll eine Reduktion des Anpressdruckes des Prothesenschaftes an die Innenwand des operativ geschaffenen Knochenhohlraumes erreicht werden. Es hat sich nun gezeigt, dass bei distal "offenen" Schäften beim Einsetzen der Prothese die Gefahr des Steckenbleibens besteht. Ausserdem hat sich erwiesen, dass besonders am offenen Ende unter Umständen eine zu starke elastische Federwirkung auftritt, wodurch Relativbewegungen zwischen Schaft und Knochen und eine Verringerung der Torsionsstabilität des Schaftes auftreten können.

Aufgabe der Erfindung ist es, die geschilderten Nachteile der bekannten Konstruktionen zu beseitigen und das Einsetzen des Schaftes in den Knochen zu erleichtern.

Diese Aufgabe wird dadurch gelöst, dass der Hohlraum oder Längsschlitz nach distal geschlossen ist.

Wie ein offener Schlitz, erlaubt auch der distal geschlossene Schlitz eine Abstufung im Anpressdruckes des Schaftes; dadurch können allmähliche Uebergänge für die Krafteinleitung in den Knochen geschaffen und übermässige Spannungsbelastungen vermieden werden.

Andererseits erlaubt der distale Abschluss des Schlitzes, die Elastizität am distalen Ende zu begrenzen, so dass die Torsionsstabilität gewahr bleibt. Weiterhin ist ein Steckenbleiben des Schaftes beim Implantieren nicht mehr zu befürchten.

Um die Elastizität des Schaftes zu beeinflussen, ist es möglich, die Schlitzbreite entsprechend der Schaftbreite von proximal nach distal zu verringern; weiterhin kann der geschlossene Längsschlitz als Depot für mindestens einen pharmazeutischen Wirkstoff dienen. Bleibt der Schlitz seitlich offen, so dass Gewebe durch ihn hindurch wächst, so können unter Umständen notwendige Reoperationen erleichtert werden, wenn der distale Rand des Längsschlitzes als Schneidkante ausgebildet ist; andererseits kann es jedoch auch vorteilhaft sein, in den Schlitz einen Kunststoff-Füllkörper einzusetzen oder den Schlitz mit zwei Blechen zu verschliessen.

Im folgenden wird die Erfindung anhand von Ausführungsbeispielen im Zusammenhang mit der Zeichnung näher erläutert.

Fig. 1 zeigt das distale Ende eines sogenannten Geradschaftes eines Femurkopfprothese;

Fig. 2 ist der Schnitt II-II von Fig. 1;

Fig. 3 gibt in gleicher Darstellung wie Fig. 1 eine weitere Ausführungsform des neuen Schaftes wieder, während

Fig. 4 den Schnitt IV-IV von Fig. 3 darstellt.

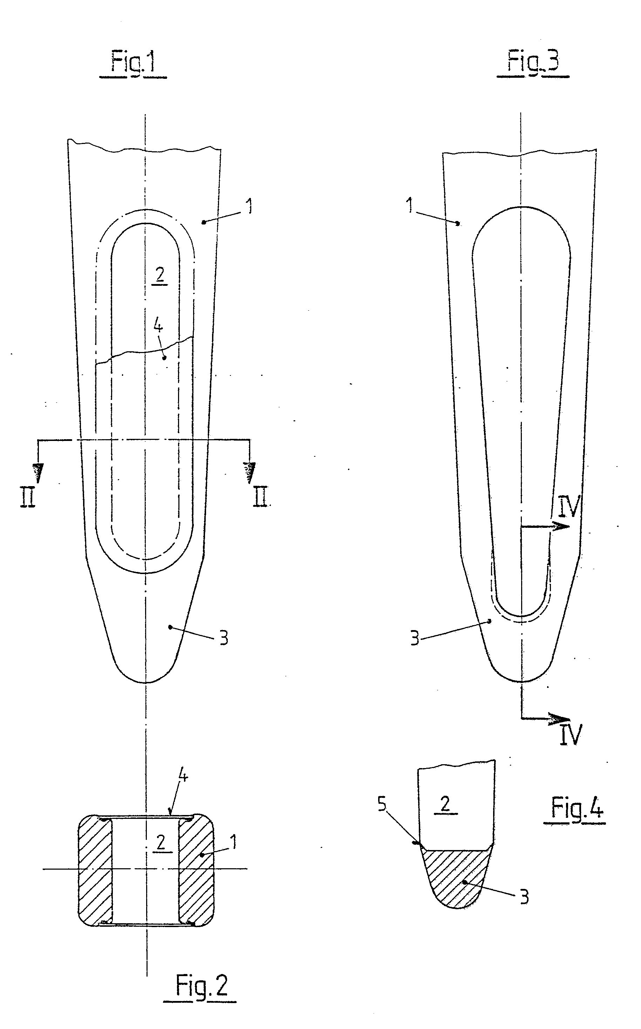

Das in Fig. 1 gezeigte distale Ende eines metallischen Femurschaftes 1 verjüngt sich nach distal allseitig konisch; es hat einen im wesentlichen rechteckigen Querschnitt (Fig. 2) und ist mit einem Hohlraum oder Schlitz 2 versehen. Nach der vorliegenden Erfindung ist der Schlitz 2 nach distal durch ein ihn überbrückendes Abschlusselement 3, das integraler Bestandteil des Schaftes 1 ist, abgeschlossen.

Der vom Schlitz 2 gebildete, geschlossene Hohlraum im "Innern" des distalen Schaftendes kann mit einem nicht gezeigten Kunststoff-Füllkörper gefüllt sein, um ein Einwachsen von Gewebe zu verhindern. Es ist jedoch auch möglich, diesen Hohlraum in bekannter Weise (siehe z.B. EP-A-244 720) als Medikamenten-Speicher zu nutzen. Der Hohlraum ist dann vorteilhafterweise mit porösen Deckeln 4 verschlossen, die ebenfalls ein Einwachsen von Gewebe verhindern, wenn durch das Herauslösen von pharmazeutischen Wirkstoffen hohle Bereiche entstehen.

Wie Fig. 3 zeigt, kann der Schlitz 2 entsprechend der konischen Verjüngung des Schaftes 1 ebenfalls nach distal verjüngt sein. Weiterhin ist es möglich, bei seitlich offenem Hohlraum oder Schlitz 2 Reoperationen durch Schneidkanten 5 am distalen Rand des Schlitzes 2 zu erleichtern, durch die eingewachsenes Gewebe beim Ausziehen des Schaftes 1 durchtrennt wird.

## Patentansprüche

1. Verankerungsschaft für eine zementfreie Verankerung einer Femurkopfprothese, dessen distaler Bereich über eine gewisse Länge hohl ausgebildet oder mit einem Längsschlitz versehen ist, dadurch gekennzeichnet, dass der Hohlraum oder Längsschlitz (2) nach distal geschlossen ist.

2. Verankerungschaft nach Anspruch 1, dadurch gekennzeichnet, dass die Schlitzbreite sich, entsprechend der Schaftbreite, von proximal nach distal verringert.

3. Verankerungsschaft nach Anspruch 1 oder 2, dadurch gekennzeichnet, dass der geschlossene Längsschlitz (2) als Depot für mindestens einen pharmazeutischen Wirkstoff dient.

4. Verankerungsschaft nach einem der Ansprüche 1 bis 3, dadurch gekennzeichnet, dass in den Längsschlitz (2) ein Kunststoff-Füllkörper eingesetzt ist.

5. Verankerungsschaft nach Anspruch 1 oder 2, dadurch gekennzeichnet, dass der distale Rand des Längsschlitzes (2) als Schneidkante (5) ausgebildet ist.

Fig.1

Fig.3

Fig.2

Fig.4

<table>
<tr><td>Europäisches Patentamt</td><td>EUROPÄISCHER RECHERCHENBERICHT</td><td>Nummer der Anmeldung</td></tr>
</table>

EP 89 81 0510

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 289 163 (SARATOVSKY NAUCHNO-ISSLEDOVATELSKY INSTITUT TRAVMATOLOGII I ORTOPEDII) * Figur 3 * | 1 | A 61 F 2/36 |
| Y | | 3 | |
| A | | 2 | |
| X | EP-A-0 190 446 (GEBRÜDER SULZER) * Figur 1; Seite 5, Zeilen 13-16 * | 1 | |
| A | | 4 | |
| X | DE-A-2 933 271 (M.A.N.) * Anspruch 1; Figur 1 * | 1 | |
| A | | 2 | |
| Y,D | EP-A-0 244 720 (GEBRÜDER SULZER) * Zusammenfassung * | 3 | |
| A | FR-A-2 591 885 (C. MAI et al.) | | |

RECHERCHIERTE SACHGEBIETE (Int. Cl.5)

A 61 F

Der vorliegende Recherchenbericht wurde für alle Patentansprüche erstellt

| Recherchenort | Abschlußdatum der Recherche | Prüfer |
|---|---|---|
| DEN HAAG | 29-09-1989 | NICE P.R. |

EPO FORM 1503 03.82 (P0403)